# EUROPÄISCHE PATENTSCHRIFT

(11) **EP 0 792 256 B1**
(45) Veröffentlichungstag und Bekanntmachung des Hinweises auf die Patenterteilung: **11.08.1999**
(21) Anmeldenummer: 95940185.2
(22) Anmeldetag: 16.11.1995
(51) Int. Cl.: C07C 45/50, C07C 29/16, C07B 35/02, B01J 35/00

(54) **VERFAHREN ZUR HERSTELLUNG VON ALDEHYDEN**
ALDEHYDE PREPARATION PROCESS
PROCEDE DE PREPARATION D'ALDEHYDES

(30) Priorität: 17.11.1994 DE 4440801
(43) Veröffentlichungstag der Anmeldung: 03.09.1997
(73) Patentinhaber: BASF AKTIENGESELLSCHAFT, 67056 Ludwigshafen (DE)
(72) Erfinder: KORMANN, Claudius, D-55411 Bingen (DE); KNEUPER, Heinz-Josef, D-68163 Mannheim (DE)
(86) Internationale Anmeldenummer: EP9504506
(87) Internationale Veröffentlichungsnummer: WO9616012

(56) Entgegenhaltungen:
- WO-A-82/03856
- DE-A- 4 230 871
- US-A- 4 400 547
- DATABASE WPI Week 9316 Derwent Publications Ltd., London, GB; AN 93-128930 & JP,A,05 064 744 (NIOD NOK CORP) , 19.März 1993

## Beschreibung

Die vorliegende Erfindung betrifft ein Verfahren zur Herstellung von Aldehyden und/oder Alkoholen durch die Hydroformylierung von Olefinen mit mehr als 3 Kohlenstoffatomen mittels eines homogen im Reaktionsmedium gelösten "nackten" Rhodiumkatalysators bei erhöhtem Druck und bei erhöhter Temperatur und Abtrennung des Rhodiumkatalysators aus dem flüssigen Reaktionsgemisch.

Die Hydroformylierung von Olefinen mit Kohlenmonoxid und Wasserstoff in Gegenwart von Übergangsmetall-Katalysatoren ist bekannt. Während α-Olefine sehr gut mit rhodiumhaltigen, phosphinmodifizierten Katalysatoren hydroformylierbar sind (vgl. J. Falbe, Ed: New Syntheses With Carbon Monoxide, Springer, Berlin 1980, S. 55 ff), ist dieses Katalysatorsystem für interne und interne, verzweigte Olefine sowie für Olefine mit mehr als 7 Kohlenstoffatomen wenig geeignet (vgl. Falbe, S. 95 ff). So werden interne Kohlenstoff-Kohlenstoff-Doppelbindungen nur sehr langsam in Gegenwart eines derartigen Katalysators hydroformyliert. Da die Abtrennung des Hydroformylierungsproduktes vom homogen im Reaktionssystem gelösten Katalysator in der Regel destillativ erfolgt und sich der Siedepunkt des bei der Hydroformylierung gebildeten Aldehyds mit zunehmender Kohlenstoffzahl und Kettenlänge auf Temperaturen erhöht, bei denen sich der rhodiumhaltige Katalysator zersetzt, ist diese Hydroformylierungsmethode für die Hydroformylierung von Olefinen mit mehr als 7 Kohlenstoffatomen nicht wirtschaftlich. Bei der Hydroformylierung polymerer Olefine, z.B. von Polyisobuten, kann der edelmetallhaltige Katalysator nicht in wiederverwendbarer Form wiedergewonnen werden.

Hingegen lassen sich interne und interne, verzweigte Olefine vorteilhaft mit sogenanntem "nacktem" Rhodium hydroformylieren, d.h. mit homogen im Hydroformylierungsmedium gelösten Rhodiumverbindungen, die nicht mit phosphorhaltigen Liganden, wie Phosphinen oder Phosphiten, modifiziert sind. Solche nicht mit Phosphinen oder Phosphiten modifizierte Rhodium-Katalysatoren und deren Eignung als Katalysator zur Hydroformylierung der zuvor genannten Klassen von Olefinen sind bekannt (siehe Falbe, S. 38 ff). Die Begriffe "nacktes Rhodium" oder "nackte Rhodium-Katalysatoren" werden in dieser Anmeldung für Rhodium-Hydroformylierungskatalysatoren gebraucht, die im Gegensatz zu herkömmlichen Rhodium-Hydroformylierungskatalysatoren unter den Bedingungen der Hydroformylierung nicht mit Liganden, insbesondere nicht mit phosphorhaltigen Liganden, wie Phosphin- oder Phosphit-Liganden, modifiziert sind. Als Liganden in diesem Sinne werden nicht Carbonyl- oder Hydrido-Liganden verstanden. Es wird in der Fachliteratur (s. Falbe, S. 38 ff) angenommen, daß die Rhodium-Verbindung HRh(CO)₄ die katalytisch aktive Rhodiumspezies bei der Hydroformylierung mit "nackten Rhodium-Katalysatoren" ist, obgleich dies aufgrund der vielen in der Hydroformylierungsreaktionszone nebeneinander ablaufenden Chemismen nicht eindeutig bewiesen ist. Lediglich der Einfachheit halber machen wir auch in dieser Anmeldung von dieser Annahme Gebrauch, ohne daß dadurch eine Einschränkung des Schutzumfangs der vorliegenden Anmeldung in Betracht zu ziehen wäre, falls sich zukünftig einmal eine andere Rhodiumspezies als die genannte, als die eigentlich katalytisch aktive, herausstellen sollte. Die "nackten Rhodium-Katalysatoren" bilden sich unter den Bedingungen der Hydroformylierungsreaktion aus Rhodium-Verbindungen, z.B. Rhodiumsalzen, wie Rhodium(III)chlorid, Rhodium(III)nitrat, Rhodium(III)acetat, Rhodium(II)acetat, Rhodium(III)sulfat oder Rhodium(III)ammoniumchlorid, aus Rhodiumchalkogeniden, wie Rhodium(III)oxid oder Rhodium(III)sulfid, aus Salzen von Rhodiumsauerstoffsäuren, beispielsweise den Rhodaten, aus Rhodium-Carbonyl-Verbindungen, wie Rh₄(CO)₁₂ und Rh₆(CO)₁₆ oder aus Organo-Rhodium-Verbindungen, wie Rhodiumdicarbonylacetonylacetonat, Cyclooctadien-Rhodiumacetat oder -chlorid in Gegenwart von CO/H₂Gemischen, die gemeinhin als Synthesegas bezeichnet werden. Zur Durchführung von Hydroformylierungen mit "nacktem" Rhodium sei an dieser Stelle beispielhaft auf die folgenden Literaturstellen verwiesen. USA 4 400 547; DE-A 33 38 340; DE-A 26 04 545; WO 82/03856; Chem. Ber. 102, 2238 (1969); Tetrahedron Lett. 29, 3261 (1968); Hydrocarbon Process. 85-86 (1975).

Allerdings hat auch die Hydroformylierung mit "nacktem" Rhodium den Nachteil, daß sich der thermolabile Rhodium-Katalysator (vgl. US-A 4 400 547) infolge der thermischen Belastung bei der destillativen Aufarbeitung des Hydroformylierungsproduktes teilweise zu metallischem Rhodium zersetzt, das sich an den Wandungen des Reaktors und der Rohrleitungen abscheidet. Das ausgefallene Rhodiummetall kann nicht wieder in die Hydroformylierungsreaktion zurückgeführt werden, da es unter den Hydroformylierungsbedingungen nicht in die katalytisch aktive Rhodiumverbindung umgewandelt werden kann. Die aus diesem chemischen Verhalten der "nackten Rhodium-Katalysatoren" resultierenden Rhodiumverluste haben bislang eine größere industrielle Anwendung dieses Verfahrens verhindert.

In DE-A 33 38 340 und USA 4 400 547 werden Verfahren zur Hydroformylierung mittels "nackter" Rhodium-Katalysatoren beschrieben, bei denen zur Verhinderung der Rhodiumabscheidung dem Reaktionsaustrag der Hydroformylierung ein Phosphin oder Phosphit zugesetzt wird, welche den Rhodium-Katalysator durch die Bildung von Phosphin- und/oder Phosphit-Komplexen vor einer thermischen Zersetzung im Zuge der destillativen Aufarbeitung des Hydroformylierungsaustrags schützen. Nach Abschluß der Destillation wird der rhodiumhaltige Destillationssumpf mit einem Oxidationsmittel behandelt, wobei das Rhodium in katalytisch aktiver Form aus den betreffenden Phosphin- oder Phosphit-Komplexen freigesetzt wird und die Phosphin- und Phosphit-Liganden zu den entsprechenden, unter Hydroformylierungsbedingungen keine Rhodium-Komplexe bildenden Phosphinoxiden und Phosphaten oxidiert werden. Der oxidierte Destillationssumpf wird dann erneut als Katalysator zur Hydroformylierung eingesetzt. Die bei der Oxidation entstandenen oxidierten Phosphorverbindungen stören bei der Hydroformylierung in der Regel nicht, jedoch reichern sich verfahrensbedingt die oxidierten Phosphorverbindungen in diesem Hydroformylierungskreislauf an, weshalb ständig ein Teilstrom dieser Katalysatorlösung aus dem Hydroformylierungskreislauf ausgeschleust und durch frische Katalysatorlösung ergänzt werden muß. Die ausgeschleuste Katalysatorlösung muß einer gesonderten Prozedur zur Wiedergewinnung des darin enthaltenen Rhodiums unterzogen werden.

WO 82/03856 betrifft ein verfahren zur Thermostabilisierung von unmodifizierten, also "nackten" Rhodium-Katalysatoren, in dem der Austrag aus der Hydroformylierungsreaktion mit einem sauerstoffhaltigen Gas behandelt wird, wodurch die gebildeten Aldehyde z.T. zu den entsprechenden Carbonsäuren oxidiert werden, welche mit dem Rhodium-Katalysator bei der destillativen Aufarbeitung thermostabile Rhodium-Carboxylate bilden, die wieder als Katalysatoren zur Hydroformylierung verwendet werden können. Nachteilig an diesem Verfahren ist die Verringerung der Ausbeute, als Folge der partiellen Oxidation der Produktaldehyde zu Carbonsäuren. Darüber hinaus ist dieses Verfahren auf solche Hydroformylierungen beschränkt, bei denen destillierbare Produkte gebildet werden: So kann nach diesem Verfahren z.B. der Rhodium-Katalysator nicht vom Hydroformylierungsprodukt des Polyisobutens abgetrennt werden.

Zur Vermeidung von Rhodiumverlusten wurde gemäß US-A 3 984 478 ein Hydroformylierungsverfahren entwickelt, bei dem die Hydroformylierung in Gegenwart eines gegebenenfalls sulfonierten Phthalocyanins durchgeführt wird. Da die hierbei gebildeten Rhodium-Phthalocyanin-Komplexe zum Teil schwer löslich oder nur in Wasser, nicht jedoch im organischen Hydroformylierungsmedium löslich sind, wird die Hydroformylierung wahlweise in Gegenwart der festen Rhodium-Phthalocyanine oder im Zwei-Phasen-System mit Wasser durchgeführt. Allerdings ist die koordinative Bindung des Rhodiums zum Phthalocyanin in diesen Komplexen sehr fest, so daß das Rhodium auch unter den Hydroformylierungsbedingungen ans Phthalocyanin gebunden bleibt. Als Folge hiervon findet die Hydroformylierungsreaktion nur an der Grenzfläche Hydroformylierungsmedium/festes Phthalocyanin bzw. an der Grenzfläche zur wäßrigen Rhodium-Phthalocyanin-Komolexlösung statt, wodurch die Reaktionsgeschwindigkeit und damit auch die Raum-Zeit-Ausbeute der Hydroformylierungsreaktion so niedrig wird, daß sich dieses Verfahren nicht wirtschaftlich betreiben läßt.

EP-A 588 225 beschreibt ein Verfahren zur extraktiven Abtrennung von Rhodium aus Hydroformylierungsausträgen mit einer wäßrigen Lösung eines wasserlöslichen, stickstoffhaltigen Chelatkomplexbildners, wobei aus dem bei der Extraktion gebildeten Rhodium-Chelatkomplex unter den Bedingungen der Hydroformylierung wieder "nacktes" Rhodium freigesetzt wird. Zur Durchführung dieses Verfahrens im technischen Maßstab werden mehrere Mixer-Settler-Apparaturen benötigt, wodurch dieses Verfahren bei der Herstellung von Aldehyden, die nur in relativ geringen Mengen benötigt werden, unkompetitiv teuer werden kann.

In der zum Zeitpunkt dieser Anmeldung noch unveröffentlichten deutschen Patentanmeldung Anmeldenummer P 43 39 139.7 wird vorgeschlagen, Rhodium-haltige Magnetpigmente als Katalysatoren für die Hydroformylierung von Olefinen einzusetzen.

Der vorliegenden Erfindung lag daher die Aufgabe zugrunde, ein Verfahren zur Hydroformylierung von Olefinen mittels eines "nackten" Rhodiumkatalysators zu finden, mit dem zum einen die Probleme der Abscheidung metallischen Rhodiums bei der destillativen Aufarbeitung des Hydroformylierungsaustrags sowie die Rhodium-Katalysatorabtrennung aus nicht destillierbaren, insbesondere hochsiedenden oder polymeren Produktaldehyden, zufriedenstellend gelöst werden können. Es sollte zu diesem Zweck nach einem Hydroformylierungsverfahren gesucht werden, bei dem die Vorläuferverbindung des "nackten" Rhodium-Katalysators an ein festes Trägermaterial gebunden ist und daraus unter dem bei der Hydroformylierungsreaktion vorliegenden CO/H₂-Druck unter Bildung des "nackten", im Reaktionsmedium der Hydroformylierungsreaktion homogen löslichen Rhodium- oder Ruthenium-Katalysators reversibel herausgelöst wird, wobei der so gebildete "nackte" Rhodium-Katalysator sich nach Beendigung der Hydroformylierungsreaktion wieder an das Trägermaterial binden sollte. Das Trägermaterial sollte dabei den Rhodium-Katalysator reversibel binden, d.h. nach Abtrennung des mit Rhodium beladenen Trägers aus dem Hydroformylierungsreaktionsgemisch sollte dieser Träger wiederholt als Rhodiumqelle für die Hydroformylierung mit "nacktem" Rhodium einsetzbar sein. Eine weitere Anforderung an den Träger bestand darin, daß er auf einfache Weise und praktisch ohne Verluste aus dem Hydroformylierungsaustrag abtrennbar sein sollte, insbesondere sollten bei der Abtrennung des edelmetallbeladenen Trägers keine aufwendigen und verlustträchtigen Filtrationsschritte erforderlich sein.

Dementsprechend wurde ein Verfahren zur Herstellung von Aldehyden und/oder Alkoholen durch die Hydroformylierung von Olefinen mit mehr als 3 Kohlenstoffatomen mittels eines homogen im Reaktonsmedium gelösten "nackten" Rhodium-Katalysators bei erhöhtem Druck und bei erhöhter Temperatur und Abtrennung des Rhodiumkatalysators aus dem flüssigen Reaktionsgemisch gefunden, das dadurch gekennzeichnet ist, daß man zur Abtrennung des homogen gelösten, "nackten" Rhodiumkatalysators aus dem Hydroformylierungsmedium ein mit einem polymeren Bindemittel beschichtetes, magnetisierbares, anorganisches Pigment verwendet und dieses Pigment, nach dessen Beladung mit dem im Hydroformylierungsmedium enthaltenen Rhodium durch Anlegen eines äußeren Magnetfeldes vom flüssigen Hydroformylierungsmedium abscheidet.

Des weiteren wurde ein Verfahren zur Herstellung von Aldehyden und/oder Alkoholen durch die Hydroformylierung von Olefinen mit mehr als 3 Kohlenstoffatomen mittels eines homogen im Reaktionsmedium gelösten, "nackten" Rhodium-Katalysators bei erhöhtem Druck und bei erhöhter Temperatur und Abtrennung des Rhodium-Katalysators aus dem Reaktionsgemisch gefunden, das dadurch gekennzeichnet ist, daß man als Rhodium-Quelle für die Erzeugung des homogen im Reaktionsmedium löslichen Rhodium-Katalysators ein unbeschichtetes oder ein mit einem polymeren Bindemittel beschichtetes, magnetisierbares, anorganisches Pigment verwendet, welches das Rhodium in unter den Bedingungen der Hydroformylierungsreaktion reversibel komplex oder adsorptiv gebundener Form enthält, einsetzt und nach Beendigung der Hydroformylierung, das wieder mit dem Rhodium beladene magnetisierbare Pigment durch Anlegen eines äußeren Magnetfeldes vom flüssigen Hydroformylierungsmedium abscheidet.

Die vorliegende Erfindung betrifft somit ein Verfahren zur Hydroformylierung von Olefinen mit einem homogen im Reaktionsmedium löslichen, "nackten" Rhodium- oder Ruthenium-Katalysator. Als Quelle für diesen homogenen Hydroformylierungskatalysator dient erfindungsgemäß ein heterogenes, anorganisches, magnetisierbares Pigment, welches den chemischen Vorläufer des "nackten" Hydroformylierungskatalysators an seiner Oberfläche gebunden trägt, wobei diese Bindung des Katalysatorvorläufers an das Pigment adsorptiv, also physikalischer Art sein kann, oder eine chemische Bindung, insbesondere eine salzartige Bindung oder eine Komplexbindung sein kann. In Abhängigkeit vom im Hydroformylierungsreaktor herrschenden CO/H₂-Druck oder im Fall der Einschaltung einer Vorcarbonylierungsstufe, in die Hydroformylierungsanlage, vom darin herrschenden Kohlenmonoxid- oder CO/H₂-Druck, wird der Katalysatorvorläufer in den "nackten" Rhodium-Katalysator umgewandelt, löst sich dabei aus der Bindung an das Pigment und geht, in homogen gelöster Form in das flüssige Hydroformylierungsmedium über, wo er seine katalytische Aktivität entfaltet, wohingegen die nun des ursprünglich daran angebundenen Katalysatorvorläufers entledigten Pigmentteilchen je nach Verfahrensausgestaltung im Hydroformylierungsmedium als katalytisch inaktive Suspension verbleiben oder vom Hydroformylierungsmedium abgetrennt werden können. Nach Beendigung der Hydroformylierungsreaktion und/oder nach Abbau des im Hydroformylierungsaustrag noch vorhandenen CO/H₂-Drucks bindet sich der "nackte" Hydroformylierungskatalysator wieder an das Magnetpigment, wobei der Katalysatorvorläufer wieder zurückgebildet wird. Das erneut mit dem Katalysatorvorläufer beladene Magnetpigment kann auf einfache Weise, beispielsweise durch Anlegen eines äußeren Magnetfeldes aus dem nun vom "nackten" Hydroformylierungskatalysator befreiten Hydroformylierungsaustrag abgeschieden werden und erneut, in einem oder mehreren derartigen Zyklen, als Rhodiumquelle für die Hydroformylierungsreaktion eingesetzt werden. Für die Durchführung des erfindungsgemäßen Verfahrens ist es somit kritisch, daß der an das magnetisierbare Pigment, im folgenden auch als Magnetpigment bezeichnet, gebundene Katalysatorvorläufer in Abhängigkeit vom CO/H₂- oder gegebenenfalls vom CO-Druck von diesem Magnetpigment unter Bildung des homogenen Katalysators reversibel abgelöst werden kann, wobei "reversibel" bedeutet, daß der homogene Katalysator bei einem geringeren CO/H₂-Druck, als dem zu seiner Ablösung vom Magnetpigment oder dem zu seiner Stabilisierung im Hydroformylierungsmedium erforderlichen CO/H₂-Druck, sich wieder, unter Rückbildung des ursprünglichen Katalysatorvorläufers oder einer anderen als Katalysatorvorläufer geeigneten Verbindung, an das Magnetpigment bindet.

Das mit der Katalysatorvorläuferverbindung beladene Magnetpigment dient somit erfindungsgemäß als helle für den unter den Reaktionsbedingungen der Hydroformylierungsreaktion zu bildenden "nackten" Rhodium- oder Rutheniumkatalysator, wohingegen das unbeladene Magnetpigment nach Beendigung der Hydroformylierungsreaktion und Aufhebung des CO/H₂-Drucks sozusagen die Funktion eines Adsorptionsmittels für den homogen gelösten Hydroformylierungskatalysator wahrnimmt, indem es den homogen gelösten Hydroformylierungskatalysator aus dem Hydroformylierungsmedium aufnimmt und bindet.

Die im erfindungsgemäßen Verfahren einsetzbaren Magnetpigmente können aus magnetisierbaren Teilchen oder aus mit einem Bindemittel beschichteten magnetisierbaren Teilchen bestehen. Unter magnetisierbaren Teilchen sind solche Teilchen zu verstehen, die in einem äußeren Magnetfeld magnetisch werden. Im allgemeinen weisen solche Stoffe eine Sättigungsmagnetisierung von 20 bis 200 nTm³/g auf, bevorzugt 30 bis 100 nTm³/g, gemessen in einem Feld von 400 kA/m. Die Größe der magnetisierbaren Teilchen ist in weiten Grenzen wählbar. Soll jedoch eine permanente Magnetisierung dieser Teilchen durch ein äußeres Magnetfeld vermieden werden, hat es sich als vorteilhaft erwiesen, Teilchen mit Durchmessern von 5 bis 1000 nm zu verwenden. Besonders bevorzugt sind Teilchen mit Durchmessern von 5 bis 100 nm. Die Größe von Teilchen mit solchen Durchmessern wird nach bekannten Methoden, z.B. durch elektronenmikroskopische Verfahren ermittelt, wobei die Werte Durchschnittswerte der jeweiligen Probe wiedergeben.

Im einzelnen kommen folgende Stoffe als magnetisierbare Kerne in Betracht; Eisen, Nickel, Cobalt, Chromdioxid, Eisenoxide sowie kubische und hexagonale Ferrite, wie mit Mangan-, Zink- und Cobaltionen sowie mit Magnesium-, Calcium-, Strontium- und Bariumionen dotierte Ferrite. Solche Stoffe sind in an sich bekannter Weise erhältlich, z.B. durch Fällungsreaktionen entsprechender Metallsalze. So kann Magnetit (Fe₃O₄) aus Lösungen von Fe²⁺/Fe³⁺-Chloriden durch Fällung mit Natronlauge hergestellt werden, z.B. gemäß DE-A 36 19 746 und Chromdioxid durch Hydrothermalsynthese, z.B. nach EP-A 27 640. Die metallischen Kerne können auch durch thermische Zersetzung von Metallcarbonylen, z.B. gemäß US-A 3 228 881, hergestellt werden. Bevorzugte Materialien für die magnetisierbaren Kerne sind Magnetit und γ-Fe₂O₃.

Entsprechend ihrer in der Regel kleinen Durchmesser weisen die magnetisierbaren Kerne im allgemeinen Oberflächen, bestimmt nach der BET-Methode (Vorschrift: DIN 66 132), von 1 bis 300 m²/g auf.

Die magnetisierbaren Kerne können direkt mit Rhodiumverbindungen umgesetzt werden, die sich adsorptiv oder chemisch an die Oberfläche des Kerns binden. Bevorzugt wird jedoch der magnetische Kern zunächst mit einem Bindemittel überzogen. Dieses Bindemittel sollte sich adsorptiv oder chemisch an den magnetisierbaren Kern binden und gleichzeitig die Möglichkeit bieten, adsorptiv oder vorzugsweise chemisch Rhodiumverbindungen zu binden, so daß diese unter den Bedingungen der Hydroformylierung vom beschichteten Magnetpigment unter Bildung des "nackten" Rhodiumkatalysators reversibel abgelöst werden.

Als Bindemittel werden vorzugsweise organische Polymere verwendet, die wasserlöslich oder in Wasser dispergierbar sind, was bedeutet, daß sich zweckmäßigerweise mindestens 1 g des betreffenden Polymers in Wasser lösen oder dispergieren lassen sollte. Zur Verbesserung der Löslichkeit oder der Dispergierbarkeit des Polymers im Wasser können dem Wasser noch bis zu 50 Vol-%, vorzugsweise bis zu 20 Vol-%, insbesondere bis zu 10 Vol-% eines wasserlöslichen organischen Lösungsmittels, wie C₁- bis C₄-Alkohole, insbesondere Methanol, Ethanol, Propanol, Isopropanol oder tert.-Butanol, wasserlösliche Ether, z.B. Tetrahydrofuran, Dioxan oder Dimethoxyethan, wasserlösliche Ketone, z.B. Aceton, wasserlösliche Amide, z.B. N,N-Dimethylformamid oder N-Methylpyrrolidon oder wasserlösliche Sulfoxide, z.B. Dimethylsulfoxid, zugemischt werden.

Bevorzugt werden solche polymeren Bindemittel zur Beschichtung der erfindungsgemäß einsetzbaren Magnetpigmente verwendet, die polare funktionelle Gruppen enthalten, beispielsweise Carboxylgruppen, Sulfonsäuregruppen, Amidgruppen, Phosphonsäuregruppen, Aminogruppen oder andere zur Ausbildung koordinativer Bindungen befähigte, stickstoffhaltige Gruppen. Diese Bindemittel können Homo- oder Copolymere sein und aus Monomeren, wie ungesättigten Carbonsäuren, z.B. Acrylsäure, Methacrylsäure und/oder Maleinsäure, ungesättigten Sulfonsäuren, z.B. Vinylsulfonsäure und/oder Styrolsulfonsäure, ungesättigten Phosphonsäuren, z.B. Vinylphosphonsäure, ungesättigten Anhydriden, z.B. Maleinsäureanhydrid, ungesättigten Aminen, z.B. Vinylamin, ungesättigten Amiden, z.B. Acrylamid und/oder Vinylpyrrolidon und/oder anderen ungesättigten, stickstoffhaltigen Monomeren, z.B. Vinylpyrrolidin, Vinylpyridin und/oder Vinylbipyridin aufgebaut sein. Diese Polymere, insbesondere die stickstoffhaltigen, polymeren Bindemittel, können partiell oxidiert sein; beispielsweise kann Polyvinylpyridin als Bindemittel verwendet werden, dessen Stickstoffatome zu 0 bis 90 %, bevorzugt zu 25 bis 75 %, oxidiert sind. Die polymeren Bindemittel können zusätzlich aus weiteren Comonomeren als den zuvor genannten aufgebaut sein, beispielsweise Ethylen, Propylen oder Styrol, vorzugsweise beträgt der Anteil der obengenannten, polare Gruppen tragenden Monomere im Polymer des Bindemittels jedoch mindestens 50 Gew.-%.

Zum Zwecke des erfindungsgemäßen Verfahrens geeignete polymere Bindemittel sind z.B. Polyacrylate, Polymethacrylate, Polyvinylpyrrolidon, Poly(2-vinylpyridin), (2-Vinylpyridin-)Styrol-Copolymere, Polyacrylsäureamide, Polyimide, Polyamide und Polyurethane.

Derartige Homo- und Copolymere sind im Handel erhältlich oder können nach an sich bekannten Methoden, z.B. durch radikalische Polymerisation, hergestellt werden. Ein besonders bevorzugtes Bindemittel sind Polyvinylpyridine, insbesondere Poly(2-vinylpyridin) und Poly(4-vinylpyridin), eines mittleren Molekulargewichts von 1 000 bis 1 000 000 Dalton. Diese Polyvinylpyridine können bis zu 20 Gew.-% an anderen Comonomeren, beispielsweise Styrol, Ethylen, Propylen im Polymeren eingebaut enthalten. Solche Polyvinylpyridine können im Handel bezogen werden.

Die Menge des auf das Magnetpigment aufzutragenden Bindemittels wird vorteilhaft so bemessen, daß sich auf den magnetisierbaren Pigmentteilchen mindestens eine Monolage des Bindemittels ausbilden kann. Im allgemeinen werden hierfür 0,1 bis 5 mg des als Bindemittel dienenden Polymers je Quadratmeter Oberfläche des Magnetpigments benötigt.

Zum Überziehen des magnetisierbaren Pigments mit dem Bindemittel wird das Magnetpigment zweckmäßigerweise in Wasser oder einer Mischung von Wasser mit einem wasserlöslichen organischen Lösungsmittel suspendiert und zu dieser Suspension das Bindemittel, vorteilhaft in Form einer Lösung oder Suspension in Wasser oder in einer Mischung aus Wasser und einem wasserlöslichen, organischen Lösungsmittel, gegeben, und diese Mischung bei Temperaturen von im allgemeinen 10 bis 100°C, im allgemeinen 10 bis 60 min lang gerührt. Es können auch kürzere oder längere Rührzeiten eingestellt werden. Das Magnetpigment wird daraufhin von der überstehenden Lösung oder Suspension abgetrennt, beispielsweise durch Filtration, Zentrifugation oder bevorzugt durch Abscheidung mittels Anlegung eines äußeren Magnetfeldes.

Damit diese so hergestellten Magnetpigmente als Quelle für den homogenen Hydroformylierungskatalysator im erfindungsgemäßen Verfahren dienen können, müssen sie erst noch mit dem Katalysatorvorläufer beladen werden. Besonders bevorzugt wird das Magnetpigment, vorzugsweise das beschichtete Magnetpigment, mit Rhodiumverbindungen beladen. Es können aber auch Vorläuferverbindungen anderer hydroformylierungsaktiver Metalle, wie Ruthenium oder Cobalt auf das Magnetpigment aufgebracht werden. Für das erfindungsgemäße Verfahren erweist sich die Aufbringung von Rhodiumverbindungen als Vorläuferverbindungen für den "nackten", homogenen Hydroformylierungskatalysator als besonders vorteilhaft.

Zur Aufbringung des Katalysatorvorläufers auf das unbeschichtete oder vorzugsweise auf das mit dem Bindemittel beschichtete Magnetpigment, können die betreffenden Metallverbindungen, also Rhodiumverbindungen, gelöst oder suspendiert in einem polaren oder unpolaren Lösungsmittel, mit dem beschichteten oder unbeschichteten Magnetpigment in Kontakt gebracht werden, wobei die Metallverbindungen adsorptiv oder bevorzugt, im Falle beschichteter Magnetpigmente, chemisch an das Magnetpigment gebunden werden.

Es sind eine Vielzahl von Metallverbindungen, insbesondere Rhodiumverbindungen zur Aufbringung als Katalysatorvorläufer auf das Magnetpigment geeignet, insbesondere Metallsalze, wie Rhodium(III)nitrat, Rhodium(III)acetat, Rhodiumhalogenide, insbesondere Rhodiumchloride, wie Rhodium(III)chlorid, Rhodium(II)acetat, Rhodium(III)sulfat oder Rhodium(III)ammoniumchlorid, Salze von Rhodiumsauerstoffsäuren, wie die Rhodate, Rhodium-Carbonyl-Verbindungen, wie Rh₄(CO)₁₂ und Rh₆(CO)₁₆ oder Organo-Rhodium-Verbindungen, wie Rhodiumdicarbonylacetonylacetonat, Cyclooctadien-Rhodiumacetat oder -chlorid, und andere Rhodium-Olefin-Komplexe. Bevorzugt werden zur erstmaligen Beladung des Magnetpigmentes wegen ihrer guten kommerziellen Verfügbarkeit Rhodiumnitrat und Rhodiumacetat verwendet. Erfindungsgemäß wird der im Hydroformylierungsmedium homogen gelöste, "nackte" Rhodiumkatalysator im Zuge der Abtrennung dieses Katalysators aus dem Hydroformylierungsgemisch auf dem Magnetpigment abgeschieden, wobei er durch Eingehen salzartiger oder koordinativer chemischer Bindungen mit den funktionellen Gruppen des Bindemittels in seiner chemischen Struktur verändert und in eine Katalysatorvorläuferverbindung zurückverwandelt wird.

Es ist selbstverständlich auch möglich, die Hydroformylierung mit "nacktem" Rhodium auf an sich herkömmliche Weise durchzuführen und die erfindungsgemäßen einsetzbaren Magnetpigmente zur Abtrennung des homogen gelösten, "nackten" Rhodiumkatalysators einzusetzen, d.h. einen Rhodium-haltigen Hydroformylierungsaustrag als Ausgangsmaterial zur Erstbeladung des Magnetpigments mit Rhodium zu verwenden. Die so erzeugten Rhodium-haltigen Magnetpigmente können dann als Rhodium-Quelle, wie beschrieben, in Hydroformylierungsreaktionen mit "nacktem" Rhodium als Katalysator weiterverwendet werden.

Als Lösungsmittel zur Aufbringung der Rhodiumverbindungen auf das Magnetpigment können je nach Art der Rhodiumverbindung Wasser oder polare oder unpolare organische Lösungsmittel verwendet werden. Wasser und polare organische Lösungsmittel, wie C₁- bis C₄-Alkohole, insbesondere Methanol, Ethanol, Propanol, Isopropanol und tert.-Butanol, wasserlösliche Ether, wie Tetrahydrofuran, Dioxan oder Dimethoxyethan, Pyridin, N-Methylpyrrolidon, Dimethylformamid, Dimethylsulfoxid, Aceton usw. oder Gemische dieser Lösungsmittel mit Wasser werden vorzugsweise zur Aufbringung von Rhodiumsalzen bei der erstmaligen Beaufschlagung des Magnetpigmentes mit salzartigen oder anderen wasserlöslichen Rhodiumverbindungen als Lösungsmittel für die Rhodiumverbindungen eingesetzt.

Unpolare organische Lösungsmittel, beispielsweise die zu hydroformylierenden Olefine, aromatische Kohlenwasserstoffe, wie Benzol, Toluol oder Xylol, dienen vorzugsweise als Lösungsmittel für unpolare, metallorganische Rhodiumverbindungen, wie Rhodium-Carbonyl-Verbindungen oder Rhodium-Olefin-Komplexen, bei der Beladung des Magnetpigments mit derartigen Rhodiumverbindungen. Erfindungsgemäß stellt das im Zuge des erfindungsgemäßen Verfahrens entstehende Hydroformylierungsgemisch, aus dem zu hydroformylierenden Olefin, den daraus gebildeten Aldehyden und deren höhermolekularen Kondensationsprodukten, praktisch das Lösungsmittel für die erneute Aufbringung des "nackten" Rhodiumkatalysators auf das Magnetpigment dar. Als Lösungsmittel können weiterhin Gemische aus hochsiedenden Kondensationsprodukten von Aldehyden, die bei der Hydroformylierung als Nebenprodukt entstehen und beispielsweise unter dem Namen Texanol® im Handel sind, dienen.

Die Aufbringung der Rhodiumverbindung auf das Magnetpigment kann z.B. durch Rühren einer Suspension des Magnetpigments in dem die jeweilige Rhodiumverbindung enthaltenden Lösungsmittel erfolgen. Dies kann vorzugsweise bei Raumtemperatur geschehen, es können dabei aber auch Temperaturen von 0°C bis zum Siedepunkt des angewandten Lösungsmittels, zweckmäßiger jedoch Temperaturen bis 100°C eingestellt werden. Zweckmäßigerweise beträgt dabei die Kontaktzeit des rhodiumhaltigen Lösungsmittels mit dem Magnetpigment ca. 5 Minuten, es kann selbstverständlich auch mit längeren Kontaktzeiten gearbeitet werden. Je nach Art des auf dem Magnetpigment vorliegenden Bindemittels und der Art und Anzahl der darin vorhandenen funktionellen Gruppen, werden, bezogen auf das Gewicht des anorganischen Kerns des Magnetpigments, im allgemeinen 0,05 bis 20 Gew.-% Rhodium, berechnet als Rh, an das Magnetpigment gebunden. Bei der Bindung an die funktionellen Gruppen des auf das Magnetpigment aufgezogenen Bindemittels erfährt die eingesetzte Rhodiumverbindung in der Regel eine chemische Umwandlung, indem es mit diesen Gruppen in eine chemische Wechselwirkung tritt. Es ist auch möglich, z.B. kolloidales Rhodium adsorptiv, also durch physikalische Wechselwirkung, auf dem unbeschichteten Magnetpigment abzuscheiden, bevorzugt werden die Rhodiumverbindungen jedoch durch chemische Wechselwirkungen an ein mit einem der genannten Bindemittel beschichteten Magnetpigment fixiert.

Die so erhaltenen, rhodiumhaltigen Magnetpigmente dienen als Rhodiumquelle im erfindungsgemäßen Hydroformylierungsverfahren. Sie sind je nach Art des verwendeten Bindemittels bis zu Temperaturen von 250°C einsetzbar und druckstabil.

Die Hydroformylierung wird im allgemeinen bei Temperaturen von 60 bis 180°C, vorzugsweise 80 bis 140°C und besonders bevorzugt 90 bis 130°C und bei einem Druck von im allgemeinen 50 bis 1 000 bar, durchgeführt. Zur Hydroformylierung wird Synthesegas als Reagenz benötigt. Unter Synthesegas werden CO/H₂-Gemische verstanden, in denen Kohlenmonoxid und Wasserstoff im allgemeinen in einem Molverhältnis von 1:5 bis 5:1, vorzugsweise von 4:6 bis 6:4, vorliegen. Die Hydroformylierung erfolgt ansonsten unter Bedingungen, wie sie üblicherweise bei Hydroformylierungen mit "nacktem" Rhodium angewandt werden und wie sie beispielsweise in der eingangs zitierten Literatur, betreffend die Hydroformylierung mit "nacktem" Rhodium, beschrieben sind. Gewünschtenfalls kann das Synthesegas noch mit unter den Reaktionsbedingungen inerten Gasen, wie Stickstoff, Argon oder gesättigten, gasförmigen Kohlenwasserstoffen, verdünnt werden.

In Abhängigkeit von den in der Hydroformylierungsstufe angewandten Druck- und Temperaturbedingungen und der Synthesegaszusammensetzung kann das Produktverhältnis Alkohol/Aldehyd im Hydroformylierungsaustrag beeinflußt werden. Beispielsweise wird bei der Hydroformylierung von Trimerpropylen bei jeweils gleichen Synthesegaszusammensetzungen - CO/H₂-Molverhältnis 50/50, 40/60 bzw. 60/40 - bei 130°C und einem Druck von 280 bar ein Aldehyd/Alkohol-Molverhältnis von jeweils 93/7 erhalten. Bei Erhöhung der Temperatur von 130°C auf 150°C verändert sich das Aldehyd/Alkohol-Molverhältnis im Hydroformylierungsaustrag in Abhängigkeit von der Synthesegaszusammensetzung - CO/H₂-Molverhältnis 50/50, 40/60 bzw. 60/40 - auf 76/24, 67/33 bzw. 82/18.

Die Hydroformylierung kann in An- oder Abwesenheit von organischen Lösungsmitteln durchgeführt werden. Besonders vorteilhaft ist die Verwendung organischer Lösungsmittel, insbesondere bei der Hydroformylierung langkettiger oder polymerer Olefine. Als Lösungsmittel können die im Hydroformylierungsverfahren üblicherweise eingesetzten Lösungsmittel benutzt werden, beispielsweise hochsiedende aromatische und aliphatische Kohlenwasserstoffe oder auch hochsiedende Aldehyd-Kondensationsprodukte, die im Zuge der Hydroformylierungsreaktion als Nebenprodukt als Folge der Kondensation der Produktaldehyde entstehen.

Wie Versuche gezeigt haben, löst sich der an das Magnetpigment gebundene Rhodium-Katalysatorvorläufer unter den Bedingungen der Hydroformylierungsreaktion vom Magnetpigment ab und geht in Form des "nackten" Rhodiumkatalysators in Lösung, wo letztgenannter die Hydroformylierungsreaktion katalysiert. Die Versuchsergebnisse deuten auch darauf hin, daß die Menge des in Lösung gehenden und während der Hydroformylierungsreaktion im Hydroformylierungsmedium vorhandenen, homogenen Rhodium-Katalysators auch von der Menge des im Hydroformylierungsmedium vorhandenen Olefins abhängig zu sein scheint. Offenbar bildet sich unter den Reaktionsbedingungen und in Abhängigkeit von der Anwesenheit und Konzentration der Reaktanten CO/H₂ und Olefin im Reaktor ein chemisches Gleichgewicht zwischen dem am Magnetpigment fixierten Katalysatorvorläufer und dem homogen im Reaktionsmedium vorliegenden Rhodiumkatalysator aus, mit der Folge, daß bei einer Verknappung der Reaktanten Wasserstoff und insbesondere Kohlenmonoxid und Olefin, beispielsweise infolge von deren Verbrauch im Zuge einer diskontinuierlichen Umsetzung oder infolge der Entspannung des Reaktors vom CO/H₂-Druck zwecks Beendigung der Umsetzung, der homogen im Reaktionsmedium vorliegende Rhodium-Katalysator sich wieder an das Magnetpigment bindet, so daß bei Beendigung der Hydroformylierung, das flüssige Hydroformylierungsgemisch praktisch rhodiumfrei ist und der während der Hydroformylierung im Reaktionsmedium homogen gelöst vorliegende "nackte" Rhodium-Katalysator wieder praktisch vollständig an das Magnetpigment gebunden ist.

Das wieder mit Rhodium beladene Magnetpigment läßt sich nach Beendigung der Umsetzung auf einfache und schonende Weise und praktisch verlustfrei vom flüssigen Hydroformylierungsaustrag durch Anlegen eines äußeren Magnetfeldes abtrennen. Dies kann beispielsweise dadurch bewerkstelligt werden, daß man das Hydroformylierungsmedium mit einem Permanentmagneten kontaktiert, an dem sich das Rhodium-beladene Magnetpigment abscheidet oder vorzugsweise durch das Einschalten eines Elektromagneten, wobei sich das rhodiumbeladene Magnetpigment am Magneten abscheidet und von diesem festgehalten wird.

Es können auch Magnetfilter zur Abtrennung des Magnetpigmentes vom Hydroformylierungsaustrag eingesetzt werden, beispielsweise ein Magnetfilter wie es in Journal of Magnetism and Magnetic Materials 85, 285 (1990) beschrieben ist.

Der mit Hilfe des Magnetpigmentes vom darin gelösten Rhodium-Katalysator befreite Hydroformylierungsaustrag kann, vorzugsweise nach der physikalischen Abtrennung des Magnetpigments, auf herkömmliche Weise, beispielsweise destillativ, aufgearbeitet werden. Das abgetrennte, Rhodium-beladene Magnetpigment kann vorteilhaft anschließend erneut als Rhodium-Quelle bei der Hydroformylierung wiederverwendet werden. Da die erfindungsgemäß als Rhodium-Quelle einsetzbaren Magnetpigmente in der Regel eine Remanenz von weniger als 40 nTm³/g, vorzugsweise von weniger als 20 nTm³/g, gemessen mit einem Schwingmagnetometer, haben, bleiben sie nach Entfernung oder Abschaltung des Magneten nicht permanent magnetisch, weshalb sie ihre feinteilige Pigmentstruktur beibehalten und nicht agglomerieren. Hierdurch wird die Rückführung und Wiederverwendung der Rhodium-beladenen Magnetpigmente in nachfolgenden Hydroformylierungen erheblich erleichtert und die Verteilung der Pigmentteilchen im Hydroformylierungsmedium als auch die Erhaltung der optimalen Oberfläche des Magnetpigments sichergestellt. Aufgrund ihrer Feinteiligkeit bleiben die Magnetpigmentteilchen während der Durchführung der Hydroformylierung im gerührten oder anderweitig, z.B. durch Durchleiten eines CO/H₂- oder Inertgasstroms, in Bewegung gehaltenen Hydroformylierungsmedium suspendiert, solange kein äußeres Magnetfeld angelegt ist. Nach Anschalten eines Magnetfeldes scheidet sich das Magnetpigment innerhalb kurzer Zeit vollständig aus der Hydroformylierungsflüssigkeit ab.

Die Menge des dem Hydroformylierungsreaktor als Rhodium-Quelle zugesetzten Rhodium-beladenen Magnetpigmentes pro Volumeneinheit Hydroformylierungsgemisch kann in weiten Grenzen variiert werden und ist von der Menge des auf dem Magnetpigment aufgebrachten Rhodiums abhängig. Es versteht sich von selbst, daß bei einer größeren Menge Magnetpigment und damit verbunden, einer größeren Menge verfügbaren Rhodium-Katalysators die Raum-Zeit-Ausbeute im Hydroformylierungsreaktor zunimmt und dies wiederum in Abhängigkeit von der Konzentration der die Ablösung des Rhodiums vom Magnetpigment und die Bildung des "nackten" Hydroformylierungskatalysators bewirkenden Hydroformylierungsreaktanten Wasserstoff, Kohlenmonoxid und Olefin. In der Regel wird das Magnetpigment dem Hydroformylierungsmedium in einer solchen Menge zugesetzt, daß pro Kilogramm zu hydroformylierendem Olefin 10 bis 1000, vorzugsweise 50 bis 200 Gew.-ppm Rhodium, berechnet als Rh, zur Verfügung stehen. Zweckmäßigerweise wird für den jeweils verwendeten Reaktor und das zu hydroformylierende Olefin die optimale Menge an zuzusetzendem Magnetpigment in einem Vorversuch optimiert.

Das erfindungsgemäße Hydroformylierungsverfahren kann diskontinuierlich, halbkontinuierlich oder kontinuierlich ausgeführt werden. Bei der diskontinuierlichen Arbeitsweise können an sich herkömmliche Hydroformylierungsreaktoren eingesetzt werden, die gewünschtenfalls an geeigneter Stelle, z.B. am Boden des Reaktors, zusätzlich mit einem Elektromagneten zur Abtrennung des Magnetpigmentes ausgestattet sein können. Nach beendeter Umsetzung läßt sich dann das Magnetpigment mit dem daran wieder fixierten Rhodium durch Einschalten des Elektromagneten von der Hydroformylierungsflüssigkeit abtrennen, die dann aus dem Reaktor ausgetragen werden kann. Die Abtrennung des Magnetpigmentes kann nach Entspannung des Reaktors oder bei dem im Reaktor vorliegenden Druck erfolgen. Bei erneuter Befüllung des Reaktors mit Reaktionsgut wird das Magnetfeld abgeschaltet und das Magnetpigment wieder in der Hydroformylierungsflüssigkeit suspendiert, so daß sich unter den Reaktionsbedingungen der Hydroformylierung daraus wieder der homogene, "nackte" Rhodium-Katalysator bilden kann.

Zur Abtrennung des Magnetpigmentes kann der das Magnetpigment suspendiert enthaltende Hydroformylierungsaustrag nach oder vor seiner Entspannung über ein Magnetfilter geleitet werden. Im Magnetfilter werden die Magnetpigmentteilchen festgehalten. Nach der Entleerung des Reaktors kann vorteilhaft neues Olefin in der umgekehrten Richtung über das Magnetfilter, nach Abschaltung des Magnetfeldes, in den Reaktor gepumpt werden und spült so das Magnetpigment in den Reaktor zurück.

Es ist auch möglich, den pigmenthaltigen Hydroformylierungsaustrag aus dem Hydroformylierungsreaktor in einen Behälter oder einen zweiten Hydroformylierungsreaktor zu entspannen, und dort das Magnetpigment mittels Anlegen eines Magnetfeldes abzutrennen. Anschließend kann das Magnetpigment mit neuem Olefin aufgeschlämmt und in den Hydroformylierungsreaktor zurückgeführt werden bzw. die folgende Hydroformylierung im zweiten Hydroformylierungsreaktor durchgeführt und dessen Hydroformylierungsaustrag in den ersten Hydroformylierungsreaktor entspannt werden. Letztere Verfahrensausgestaltung läßt sich auch kontinuierlich betreiben.

Das erfindungsgemäße Verfahren eignet sich besonders gut zur Hydroformylierung von Olefinen mit mehr als 3, vorzugsweise mehr als 7 Kohlenstoffatomen, insbesondere zur Hydroformylierung von C₇- bis C₂₀-Olefinen, die geradkettig oder verzweigt sein können und die α-olefinische und/oder interne Doppelbindungen enthalten können, z.B. Octen-1, Dodecen-1, Trimer- und Tetramerpropylen, oder Dimer-, Trimer- und Tetramerbutylen. Ebenso können ungesättigte Oligomere anderer Olefine hydroformyliert werden. Desgleichen Cooligomere verschiedener Olefine. Die aus diesen Olefinen gebildeten Aldehyde dienen z.B. als Vorstufen für die Herstellung von Weichmacheralkoholen und Tensiden, die daraus auf an sich herkömmliche Weise durch Hydrierung erzeugt werden können. Die zur Hydroformylierung eingesetzten Olefine können z.B. durch die säurekatalysierte Wasserabspaltung aus den entsprechenden-Fettalkoholen oder nach einer Vielzahl anderer technischer Verfahren, wie sie beispielsweise in Weissermel, Arpe: Industrielle Organische Chemie, S. 67-86, Verlag Chemie, Weinheim, 1978, dargestellt sind, erhalten werden.

Besonders gut geeignet ist das erfindungsgemäße Verfahren auch zur Hydroformylierung von polymeren Olefinen, beispielsweise niedermolekularem Polyisobuten, niedermolekularem Polybutadien oder niedermolekularem 1,3-Butadien-Isobuten- oder Buten-Copolymeren. Unter "niedermolekulare Polymere" werden insbesondere Polymere mit Molgewichten von 500 bis 5 000 Dalton verstanden. Es können aber auch höhermolekulare, ungesättigte Polymere hydroformyliert werden. Einzige Voraussetzung hierfür ist, daß diese im Hydroformylierungsmedium löslich sind. Die Hydroformylierungsprodukte dieser polymeren Olefine, insbesondere die des niedermolekularen Polyisobutens können beispielsweise nach dem Verfahren der EP-A 244 616 durch reduktive Aminierung in die entsprechenden Amine umgewandelt werden, die als Kraftstoffadditiv Verwendung finden. Niedermolekulares Polyisobuten ist beispielsweise nach dem Verfahren von EP-A 145 235 erhältllich, niedermolekulare Isobuten-1,3-Butadien-Copolymere können z.B. nach dem Verfahren der Patentanmeldung WO 94/20554 gewonnen werden.

Das erfindungsgemäße Verfahren eignet sich praktisch zur Herstellung aller Aldehyde, die auf dem Wege der Hydroformylierung von Olefinen erhältlich sind. Insbesondere sei darauf hingewiesen, daß beispielsweise auch substituierte Olefine, die im allgemeinen 1 bis 2, vorzugsweise einen Substituenten, tragen können, nach dem erfindungsgemäßen Verfahren hydroformyliert werden können. Beispielsweise können nach dem erfindungsgemäßen Verfahren ungesättigte, aliphatische Carbonsäureester, Acetale, Alkohole, Ether, Aldehyde, Ketone und Amine und Amide hydroformyliert werden . Als derartige substituierte Ausgangsolefine sind z.B. Methacrylsäureester, Dicyclopentadien, Vinyl- und Allylether, insbesondere entsprechend substituierte Derivate ungesättigter Fettsäuren von Interesse, beispielsweise die Ester der Öl-, Linol-, Linolen-, Ricinol- oder Erucasäure. Die aus diesen olefinischen Rohstoffen durch Hydroformylierung erhältlichen Aldehyde sind ebenfalls Ausgangsmaterialien zur Herstellung biologisch leicht abbaubarer, waschaktiver Substanzen.

Wie bereits erwähnt, entsteht als Hydroformylierungsprodukt ein Gemisch aus dem vom eingesetzten Olefin durch Hydroformylierung abgeleiteten Aldehyd und dem entsprechenden, infolge der Hydrieraktivität des Rhodium-Katalysators, daraus gebildeten Alkohol. Je nach Wunsch kann der Alkoholanteil im Hydroformylierungsprodukt durch die oben geschilderten Änderungen der Reaktionsbedingungen erhöht werden. Die so gebildeten Alkohole dienen ebenfalls als Weichmacher oder Tenside oder als Ausgangamaterial zur Herstellung dieser Produkte.

### Beispiele

### A. Herstellung der magnetisierbaren Teilchen

Das Magnetit wurde gemäß DE-A 35 00 471 durch eine Fällungsreaktion hergestellt, indem eine stöchiometrische Lösung von Fe⁽²⁺⁾/Fe⁽³⁺⁾-Chloriden in Wasser zu einer Lösung von Natronlauge in Wasser getropft wurde. Der Fällungsmagnetit wurde filtriert und chloridfrei gewaschen. Es entstand ein Filterkuchen mit einem Magnetitgehalt von 24 Gew.-%. Das getrocknete Pigment wurde durch folgende Meßwerte charakterisiert: Die BET-Oberfläche wurde gemäß DIN 66 132 gemessen. Sie betrug 59 m²/g. Die magnetischen Eigenschaften wurden mit einem Schwingmagnetometer ermittelt. Die Sättigungsmagnetisierung betrug 79 nTm³/g, gemessen in einem Feld von 400 kA/m.

### B. Beschichtung des Magnetit mit Polymer

Es wurde eine Lösung von 10 g Poly-4-vinylpyridin/Poly-4-vinylpyridin-N-Oxid mit einem Oxidationsgrad von 67 % und einem K-Wert (1%ig in NaCl 5%ig; bestimmt nach H. Fikentscher, Cellulose-Chemie 13, 58-64, 71-74 (1932)) von 14,2 (hergestellt gemäß WO 94/20549) in 250 g Wasser durch Zugabe von 37 g 5%iger NaOH-Lösung auf pH 9,6 gebracht. Diese Polymerlösung wurde zu 100 g des in Beispiel A beschriebenen Filterkuchens gegeben, untergerührt, mit weiteren 400 g Wasser versetzt und sodann eine Stunde lang mit einem Ultra-Turrax® Dispergieraggregat (Hersteller: Firma Janke & Kunkel, Staufen/Breisgau, BRD) unter Stickstoffabdeckung und Eiskühlung homogenisiert. In der Mischung wurde ein pH 6,5 gemessen, der durch Zugabe von 49 g 5%iger NaOH-Lösung auf pH 7,6 erhöht wurde.

### C. Herstellung des erfindungsgemäßen Katalysators

Zur unter B. beschriebenen Suspension wurde eine Lösung von 280 mg Rh(NO₃)₃ in 30 g Wasser gegeben. Es wurde weitere 15 Minuten lang dispergiert. Es wurde ein pH von 7,3 gemessen. Die Suspension wurde sodann bei 80°C am Rotationsverdampfer und am Wasserstrahlvakuum getrocknet und zuletzt zu Staub zerrieben. Der magnetische Staub wurde untersucht: Die BET-Oberfläche betrug 30 m²/g. Die Sättigungsmagnetisierung, gemessen in einem Feld von 400 kA/m, betrug 79 nTm³/g.

### D. Überprüfung der katalytischen Eigenschaften

In einem Autoklav wurden 100 g Octen N und 11 g des nach Beispiel C erhaltenen Rhodium-Polymer-Magnetit-Katalysators (entsprechend 100 ppm Rh, bezogen auf das Olefin) mit 100 bar CO/H₂-Druck (CO/H₂-Molverhältnis 1:1) beaufschlagt. Anschließend wurde auf 130°C hochgeheizt und der CO/H₂-Druck auf 280 bar erhöht. Wahrend 3 Stunden wurden Druck und Temperatur konstant gehalten. Nach der Abkühlung auf Raumtemperatur und Entspannung des Autoklaven wurde das Magnetpigment auf 2 Magnetstäben (Vacodym® 370 HR; Hersteller: Vacuumschmelze GmbH) abgeschieden. Das pigmentfreie Produktgemisch wurde analysiert. Es wurde die Bildung von Aldehyd mit einer Ausbeute von 33 % beobachtet. Die Rhodium-Konzentration im pigmentfreien Produktgemisch wurde mittels der Atomabsorptionsspektrometrie bestimmt. Sie betrug weniger als 0,1 Gew.-ppm.

### E. Wiederholte Überprüfung der katalytischen Eigenschaften

Das auf den Magnetstäben abgeschiedene feuchte Pigment des Beispiels D wurde durch Abdekantieren vom flüssigen Reaktionsprodukt abgetrennt und nach Entfernung des Magneten erneut mit 100 g Octen N versetzt. Die Hydroformylierung wurde wie in Beispiel D wiederholt. Nach der Entspannung des Autoklaven wurde das Magnetpigment wieder auf zwei Magnetstäben abgeschieden. Das pigmentfreie Produktgemisch wurde analysiert. Es wurde die Bildung von Aldehyd mit einer Ausbeute von 29 % beobachtet. Die Rhodium-Konzentration im pigmentfreien Produktgemisch betrug weniger als 0,1 Gew.-ppm.

### F. Wiederholte Überprüfung der katalytischen Eigenschaften

Das auf den Magnetstäben abgeschiedene feuchte Pigment des Beispiels E wurde durch Abdekantieren vom flüssigen Reaktionsprodukt abgetrennt und nach Entfernung des Magneten erneut mit 100 g Octen N versetzt. Die Hydroformylierung wurde wie in Beispiel D wiederholt. Nach der Entspannung des Autoklaven wurde das Magnetpigment wieder auf den Magnetstäben abgeschieden. Das pigmentfreie Produktgemisch wurde analysiert. Es wurde die Bildung von Aldehyd mit einer Ausbeute von 34 % beobachtet. Im pigmentfreien Produktgemisch wurde eine Rhodium-Konzentration von 0,1 Gew.-ppm gemessen.

### G. Herstellung von trockenem polymerbeschichtetem Magnetit

Es wurde eine Suspension wie in Beispiel B hergestellt und bei 80°C am Rotationsverdampfer und am Wasserstrahlvakuum getrocknet und zu Staub zerrieben. Der magnetische Staub wurde eine Woche lang bei 120°C an Luft erhitzt und anschließend untersucht: Die BET-Oberfläche betrug 37 m²/g, was darauf hindeutet, daß keine Verschmelzung des Pigments stattgefunden hat.

### H. Herstellung einer Rhodium-haltigen organischen Lösung

In einem Autoklav wurden 1 023 g Octen N und 530 mg Rhodium(I)dicarbonylacetylactonat (entsprechend 200 ppm Rh, bezogen auf das Olefin) mit 100 bar CO/H₂-Druck (CO/H₂-Molverhältnis 1:1) beaufschlagt. Anschließend wurde auf 130°C hochgeheizt und der CO/H₂-Druck auf 280 bar erhöht. Während 4 Stunden wurden Druck und Temperatur konstant gehalten. Nach der Abkühlung auf Raumtemperatur und der Entspannung des Autoklaven wurde die Rhodium-Konzentration im Produktgemisch gemessen: Sie betrug 130 Gew.-ppm.

### I. Herstellung des erfindungsgemäßen Katalysators

Zur Herstellung des erfindungsgemäßen Katalysators wurden 857 g der Lösung aus Beispiel H auf 80°C erhitzt. Es wurden 80 g Magnetpigment aus Beispiel G hinzugefügt und 3 Stunden lang bei 80°C gerührt. Nach dem Abkühlen wurde das Magnetpigment auf zwei Magnetstäben abgeschieden. Die überstehende Lösung wurde abdekantiert und untersucht: die Rhodium-Konzentration betrug 3 Gew.-ppm, was auf eine über 97%ige Abtrennung des Rhodiums hindeutet. Das feuchte Magnetpigment wurde bei 130 bis 150°C am Vakuum von Lösungsmittelresten befreit und in einer Schlagmühle zu Staub gemahlen. Der Staub wurde untersucht: die BET-Oberfläche betrug 32 m²/g, die Sättigungsmagnetisierung betug 65 nTm³/g, gemessen in einem Feld von 400 kA/m, sein Rhodiumgehalt betrug 1 400 Gew.-ppm.

### J. Überprüfung der katalytischen Eigenschaften

In einem Autoklav wurden 100 g Octen N und 7,1 g des nach Beispiel I erhaltenen Rhodium-Polymer-Magnetit-Katalysators (entsprechend 100 ppm Rh, bezogen auf das Olefin) mit 100 bar CO/H₂-Druck (im Molverhältnis 1:1) beaufschlagt. Anschließend wurde auf 130°C hochgeheizt und der CO/H₂-Druck auf 280 bar erhöht. Wahrend 3 Stunden wurden Druck und Temperatur konstant gehalten. Nach der Abkühlung auf Raumtemperatur und Entspannung des Autoklaven wurde das Magnetpigment auf 2 Magnetstäben (Vacodym® 370 HR) abgeschieden. Das pigmentfreie Produktgemisch wurde analysiert. Es wurde die Bildung von Aldehyd mit einer Ausbeute von 58 % beobachtet. Die Rhodium-Konzentration im pigmentfreien Produktgemisch betrug 0,5 Gew.-ppm.

### K. Wiederholte Überprüfung der katalytischen Eigenschaften

Das auf den Magnetstäben abgeschiedene feuchte Pigment des Beispiels J wurde durch Abdekantieren vom flüssigen Reaktionsprodukt abgetrennt und nach Entfernung des Magneten erneut mit 100 g Octen N versetzt. Die Hydroformylierung wurde wie in Beispiel J wiederholt. Nach der Abkühlung auf Raumtemperatur und der Entspannung des Autoklaven wurde das Magnetpigment wieder auf zwei Magnetstäben abgeschieden. Das pigmentfreie Produktgemisch wurde analysiert. Es wurde die Bildung von Aldehyd mit einer Ausbeute von 59 % beobachtet. Die Rhodium-Konzentration im pigmentfrein Produktgemisch betrug 0,3 Gew.-ppm.

### L. Wiederholte Überprüfung der katalytischen Eigenschaften

Das auf den Magnetstäben abgeschiedene feuchte Pigment des Beispiels K wurde durch Abdekantieren vom flüssigen Reaktionsprodukt abgetrennt und nach Entfernung des Magneten erneut mit 100 g Octen N versetzt. Die Hydroformylierung wurde wie in Beispiel J wiederholt. Nach der Abkühlung auf Raumtemperatur und Entspannung des Autoklaven wurde das Magnetpigment wieder auf den Magnetstäben abgeschieden. Das pigmentfreie Produktgemisch wurde analysiert. Es wurde die Bildung von Aldehyd mit einer Ausbeute von 67 % beobachtet. Im pigmentfreien Produktgemisch wurde eine Rhodium-Konzentration von 0,3 Gew.-ppm gemessen.

### M. Überprüfung der katalytischen Eigenschaften

In einem Autoklav wurden 130 g eines Gemischs von Polyisobuten (mittleres Molekulargewicht ca. 1 000) im Gemisch mit einer C₁₀- bis C₁₅-Paraffinmischung, Volumen-Verhältnis 1:1 (BASF-Handelsprodukt Glissopal® ES 3250) und 5 g des nach Beispiel I erhaltenen Rhodium-Polymer-Magnetit-Katalysators (entsprechend 52 Gew.-ppm Rh, bezogen auf das Gemisch) mit 100 bar CO/H₂-Druck (CO/H₂-Molverhältnis 1:1) beaufschlagt. Anschließend wurde auf 130°C hochgeheizt und der CO/H₂-Druck auf 280 bar erhöht. Während 5 Stunden wurden Druck und Temperatur konstant gehalten. Nach der Abkühlung auf Raumtemperatur und Entspannung des Autoklaven wurde das Magnetpigment auf 2 Magnetstäben (Vacodym® 370 HR) abgeschieden. Das pigmentfreie Produktgemisch wurde durch Destillation bei 150°C und einem Druck von 13 mbar vom Paraffinlösungsmittel befreit und sodann analysiert: Die Rhodium-Konzentration betrug 3 Gew.-ppm. Aus der CO-Zahl von 17 mg je g wurde ein Umsatz von 31 % berechnet.

### N. Beschichtung von Magnetit mit Polymer

Es wurden 237 g Filterkuchen mit 100 g Magnetit wie in Beispiel A mit einer Lösung von 10 g Vinylpyrrolidon/Vinylimidazol-Copolymer (50/50, K-Wert = 17,6) in 500 g Wasser versetzt und eine Stunde lang mit einem Ultra Turrax® Dispergieraggregat unter Stickstoffabdeckung und Eiskühlung homogenisiert. In der Mischung wurde nach Zugabe von 8 g 5%iger NaOH-Lösung ein pH 7,3 gemessen. Die Suspension wurde bei 90°C am Rotationsverdampfer und am Wasserstrahlvakuum getrocknet und zuletzt zu Staub zerrieben. Der magnetische Staub wurde untersucht: Die BET-Oberfläche betrug 44 m²/g. Die Sättigungsmagnetisierung, gemessen in einem Feld von 400 kA/m, betrug 79 nTm³/g, die Remanenz 7 nTm³/g.

### O. Herstellung des erfindungsgemäßen Katalysators

Es wurde gemäß Beispiel H eine Rhodium-haltige organische Lösung durch Verringerung der Rhodiummenge so hergestellt, daß die Rhodiumkonzentration 70 ppm betrug. 200 g dieser Lösung wurden mit 10 g Magnetstaub aus Beispiel N eine Stunde lang bei Raumtemperatur (d.h. 23 ± 2°C) gerührt. Der Magnetstaub wurde an einem Stabmagneten abgeschieden und die Rhodiumkonzentration in der überstehenden klaren Lösung wurde gemessen. Sie betrug nur noch 7 Gew.-ppm, woraus eine Abscheidung von 12,6 mg Rhodium auf dem Magnetpigment abgeleitet wurde.

Bestimmung der CO-Zahl:
Die CO-Zahl wurde alkalimetrisch bestimmt. Durchführung: Die in Toluol gelöste Probe wurde eine Stunde bei 85°C und bei einem pH-Wert von 2,5 mit einer wäßrigen Hydroxylaminhydrochlorid-Lösung oximiert. Definitionsgemäß wird dabei angenommen, daß pro Carbonylgruppe (C=O) -Gruppe eine Molekül Salzsäure freigesetzt wird. Nach dem Erkalten wurde die Lösung mit einer NaOH-Maßlösung potentiometrisch titriert.

Octen N:
Produkt der Butendimerisierung nach dem IFP-"Dimersol"-Prozeß (vgl. Weissermel, Arpe: Industrielle Organische Chemie; 2. Auflage, S. 82, Verlag Chemie, Weinheim 1978).

## Patentansprüche

1. Verfahren zur Herstellung von Aldehyden und/oder Alkoholen durch die Hydroformylierung von Olefinen mit mehr als 3 Kohlenstoffatomen mittels eines homogen im Reaktionsmedium gelösten "nackten" Rhodiumkatalysators bei erhöhtem Druck und bei erhöhter Temperatur und Abtrennung des Rhodiumkatalysators aus dem flüssigen Reaktionsgemisch, dadurch gekennzeichnet, daß man zur Abtrennung des homogen gelösten, "nackten" Rhodiumkatalysators aus dem Hydroformylierungsmedium ein mit einem polymeren Bindemittel beschichtetes, magnetisierbares, anorganisches Pigment verwendet und dieses Pigment, nach dessen Beladung mit dem im Hydroformylierungsmedium enthaltenen Rhodium, durch Anlegen eines äußeren Magnetfeldes vom flüssigen Hydroformylierungsmedium abscheidet.

2. Verfahren nach Anspruch 1, dadurch gekennzeichnet, daß man als magnetisierbares Pigment ein magnetisierbares Pigment mit einer Remanenz von weniger als 40 nTm³ /g verwendet.

3. Verfahren nach den Ansprüchen 1 und 2, dadurch gekennzeichnet, daß man als magnetisierbares Pigment feinteiliges Eisen, Nickel, Cobalt, Chromdioxid, Eisenoxide, kubische oder hexagonale Ferrite oder mit Mangan-, Zink-, Cobalt-, Magnesium-, Calcium-, Strontium- und/oder Bariumionen dotierte Ferrite verwendet.

4. Verfahren nach den Ansprüchen 1 bis 3, dadurch gekennzeichnet, daß man ein magnetisierbares Pigment verwendet, das mit einem Carboxyl-, Sulfonsäure-, Phosphonsäure-, Amid-, Amino- oder andere zur Koordination mit Rhodiumverbindungen befähigte stickstoffhaltige Gruppen enthaltenden polymeren Bindemittel beschichtet worden ist.

5. Verfahren nach Anspruch 4, dadurch gekennzeichnet, daß man ein magnetisierbares Pigment verwendet, das mit einem Polyacrylat, Polymethacrylat, Polyacrylamid, Polyvinylpyrrolidon, Polyvinylpyridin, partiell oxidiertem Polyvinylpyridin oder einem Vinylpyrrolidon/Vinylimidazol-Copolymer als polymerem Bindemittel beschichtetworden ist.

6. Verfahren zur Herstellung von Aldehyden und/oder Alkoholen durch die Hydroformylierung von Olefinen mit mehr als 3 Kohlenstoffatomen mittels eines homogen im Reaktionsmedium gelösten, "nackten" Rhodium-Katalysators bei erhöhtem-Druck und bei erhöhter Temperatur und Abtrennung des Rhodium-Katalysators aus dem Reaktionsgemisch, dadurch gekennzeichnet, daß man als Rhodium-Quelle für die Erzeugung des homogen im Reaktionsmedium löslichen Rhodium-Katalysators ein unbeschichtetes oder ein mit einem polymeren Bindemittel beschichtetes, magnetisierbares, anorganisches Pigment verwendet, welches das Rhodium in unter den Bedingungen der Hydroformylierungsreaktion reversibel komplex oder adsorptiv gebundener Form enthält, einsetzt und nach Beendigung der Hydrofornylierung, das wieder mit dem Rhodium beladene magnetisierbare Pigment durch Anlegen eines äußeren Magnetfeldes vom flüssigen Hydroformylierungsmedium abscheidet.

7. Verfahren nach Anspruch 6, dadurch gekennzeichnet, daß man ein mit einem polymeren Bindemittel beschichtetes, magnetisierbares, anorganisches Pigment verwendet.

8. Verfahren nach den Ansprüchen 6 und 7, dadurch gekennzeichnet, daß man als magnetisierbares Pigment ein magnetisierbares Pigment mit einer Remanenz von weniger als 40 nTm³/g verwendet.

9. Verfahren nach den Ansprüchen 6 bis 8, dadurch gekennzeichnet, daß man als magnetisierbares Pigment feinteiliges Eisen, Nickel, Cobalt, Chromdioxid, Eisenoxide, kubische oder hexagonale Ferrite oder mit Mangan-, Zink-, Cobalt-, Magnesium-, Calcium-, Strontium- und/oder Bariumionen dotierte Ferrite verwendet.

10. Verfahren nach den Ansprüchen 6 bis 9, dadurch gekennzeichnet, daß man ein magnetisierbares Pigment verwendet, das mit einem Carboxyl-, Sulfonsäure-, Phosphonsäure-, Amid-, Amino- oder andere zur Koordination mit Rhodiumverbindungen befähigte, stickstoffhaltige Gruppen enthaltenden polymeren Bindemittel beschichtet worden ist.

11. Verfahren nach den Ansprüchen 6 bis 10, dadurch gekennzeichnet, daß man ein magnetisierbares Pigment verwendet, das mit einem Polyacrylat, Polymethacrylat, Polyacrylamid, Polyvinylpyrrolidon, Polyvinylpyridin, partiell oxidierten Polyvinylpyridin oder einem Vinylpyrrolidon/Vinylimidazol-Copolymer beschichtet worden ist.

## Claims

1. A process for the preparation of aldehydes or alcohols by the hydroformylation of olefins of more than 3 carbon atoms by means of a bare rhodium catalyst dissolved homogeneously in the reaction medium, at superatmospheric pressure and at elevated temperatures, and separation of the rhodium catalyst from the liquid reaction mixture, wherein a magnetizable, inorganic pigment coated with a polymeric binder is used for separating the homogeneously dissolved, bare rhodium catalyst from the hydroformylation medium, and this pigment, after it has been laden with the rhodium contained in the hydroformylation medium, is separated from the liquid hydroformylation medium by applying an external magnetic field.

2. A process as claimed in claim 1, wherein the magnetizable pigment used is a magnetizable pigment having a residual induction of less than 40 nTm³/g.

3. A process as claimed in claims 1 and 2, wherein the magnetizable pigment used is finely divided iron, nickel, cobalt, chromium dioxide, iron oxides, cubic or hexagonal ferrites or ferrites doped with manganese, zinc, cobalt, magnesium, calcium, strontium or barium ions.

4. A process as claimed in any of claims 1 to 3, wherein the magnetizable pigment used is one which has been coated with a polymeric binder containing carboxyl, sulfo, phospho, amido or amino groups or other nitrogen-containing groups capable of coordination with rhodium compounds.

5. A process as claimed in claim 4, wherein the magnetizable pigment used is one which has been coated with a polyacrylate, polymethacrylate, polyacrylamide, polyvinylpyrrolidone, polyvinylpyridine, partially oxidized polyvinylpyridine or vinylpyrrolidone/vinylimidazole copolymer as the polymeric binder.

6. A process for the preparation of aldehydes or alcohols by the hydroformylation of olefins of more than 3 carbon atoms by means of a bare rhodium catalyst dissolved homogeneously in the reaction medium, at superatmospheric pressure and at elevated temperatures, and separation of the rhodium catalyst from the reaction mixture, wherein the rhodium source used for producing the rhodium catalyst which is homogeneously soluble in the reaction medium is a magnetizable, inorganic pigment which is uncoated or coated with a polymeric binder and contains the rhodium in a reversibly complexed or adsorptively bound form under the conditions of the hydroformylation reaction, and, after the hydroformylation, the magnetizable pigment again laden with the rhodium is separated from the liquid hydroformylation medium by applying an external magnetic field.

7. A process as claimed in claim 6, wherein a magnetizable, inorganic pigment coated with a polymeric binder is used.

8. A process as claimed in claims 6 and 7, wherein the magnetizable pigment used is a magnetizable pigment having a residual induction of less than 40 nTm³/g.

9. A process as claimed in any of claims 6 to 8, wherein the magnetizable pigment used is finely divided iron, nickel, cobalt, chromium dioxide, iron oxide, cubic or hexagonal ferrite or ferrite doped with manganese, zinc, cobalt, magnesium, calcium, strontium or barium ions.

10. A process as claimed in any of claims 6 to 9, wherein the magnetizable pigment used is one which has been coated with a polymeric binder containing carboxyl, sulfo, phospho, amido or amino groups or other nitrogen-containing groups capable of coordination with rhodium compounds.

11. A process as claimed in any of claims 6 to 10, wherein the magnetizable pigment used is one which has been coated with a polyacrylate, polymethacrylate, polyacrylamide, polyvinylpyrrolidone, polyvinylpyridine, partially oxidized polyvinylpyridine or vinylpyrrolidone/vinylimidazole copolymer.

## Revendications

1. Procédé de préparation d'aldéhydes et/ou d'alcools par hydroformylation d'oléfines ayant plus de 3 atomes de carbone au moyen d'un catalyseur au rhodium 'nu' dissous de façon homogène dans le milieu réactionnel, sous pression élevée et à température élevée, et séparation du catalyseur au rhodium du milieu réactionnel liquide, caractérisé en ce que l'on utilise un pigment inorganique magnétisable enduit d'un liant polymère pour la séparation du catalyseur au rhodium 'nu' dissous de façon homogène du milieu d'hydroformylation, et on sépare ce pigment du milieu liquide d'hydroformylation, par la mise en place d'un champ magnétique extérieur, après qu'il ait été chargé avec le rhodium contenu dans le milieu d'hydroformylation.

2. Procédé selon la revendication 1, caractérisé en ce que l'on utilise en tant que pigment magnétisable un pigment magnétisable ayant une rémanence de moins de 40 nTm³/g.

3. Procédé selon les revendications 1 et 2, caractérisé en ce que l'on utilise en tant que pigment magnétisable du fer finement divisé, du nickel, du cobalt, du dioxyde de chrome, de l'onde de fer, de la ferrite cubique ou hexagonale ou de la ferrite dopée avec des ions manganèse, zinc, cobalt, magnésium, calcium, strontium et/ou baryum.

4. Procédé selon l'une quelconque des revendications 1 à 3, caractérisé en ce que l'on utilise un pigment magnétisable enduit d'un liant polymère contenant des groupements carboxy, acide sulfonique, acide phosphonique, amide, amino ou d'autres groupements azotés pouvant être coordinés avec les composés du rhodium.

5. Procédé selon la revendication 4, caractérisé en ce que l'on utilise un pigment magnétisable enduit, en tant que liant polymère, avec un polyacrylate, un polyméthacrylate, un polyacrylamide, une polyvinylpyrrolidone, une polyvinylpyridine, une polyvinylpyridine partiellement oxydée ou un copolymère vinylpyrrolidone/vinylimidazole.

6. Procédé de préparation d'aldéhydes et/ou d'alcools par hydroformylation d'oléfines ayant plus de 3 atomes de carbone au moyen d'un catalyseur au rhodium 'nu' dissous de façon homogène dans le milieu réactionnel, sous pression élevée et à température élevée, et séparation du catalyseur au rhodium du milieu réactionnel, caractérisé en ce que l'on utilise en tant que source de rhodium pour l'obtention du catalyseur au rhodium dissous de façon homogène dans le milieu réactionnel, un pigment inorganique magnétisable non enduit ou enduit d'un liant polymère, qui contient le rhodium sous forme liée par adsorption ou sous forme d'un complexe réversible dans les conditions de la réaction d'hydroformylation, et, après la fin de l'hydroformylation, que l'on sépare du milieu liquide d'hydroformylation avec le pigment magnétisable chargé avec le rhodium, par la mise en place d'un champ magnétique extérieur.

7. Procédé selon la revendication 6, caractérisé en ce que l'on utilise un pigment inorganique magnétisable enduit avec un liant polymère.

8. Procédé selon les revendications 6 et 7, caractérisé en ce que l'on utilise en tant que pigment magnétisable un pigment magnétisable ayant une rémanence de moins de 40 nTm³/g.

9. Procédé selon l'une quelconque des revendications 6 à 8, caractérisé en ce que l'on utilise en tant que pigment magnétisable du fer finement divisé, du nickel, du cobalt, du dioxyde de chrome, de l'oxyde de fer, de la ferrite cubique ou hexagonale ou de la ferrite dopée avec des ions manganèse, zinc, cobalt, magnésium, calcium, strontium et/ou baryum.

10. Procédé selon l'une quelconque des revendications 6 à 9, caractérisé en ce que l'on utilise un pigment magnétisable enduit d'un liant polymère contenant des groupements carboxy, acide sulfonique, acide phosphonique, amide, amino ou d'autres groupements azotés pouvant être coordinés avec les composés du rhodium.

11. Procédé selon l'une quelconque des revendications 6 à 10, caractérisé en ce que l'on utilise un pigment magnétisable enduit, en tant que liant polymère, avec un polyacrylate, un polyméthacrylate, un polyacrylamide, une polyvinylpyrrolidone, une polyvinylpyridine, une polyvinylpyridine partiellement oxydée ou un copolymère vinylpyrrolidone/vinylimidazole.
